(19) Europäisches Patentamt
European Patent Office
Office européen des brevets

(11) Numéro de publication : **0 308 325 B1**

(12) # FASCICULE DE BREVET EUROPEEN

(45) Date de publication du fascicule du brevet : **11.12.91 Bulletin 91/50**

(51) Int. Cl.$^5$ : **C07C 255/03**, C07C 253/00, B01J 23/02

(21) Numéro de dépôt : **88402314.4**

(22) Date de dépôt : **14.09.88**

(54) **Procédé de préparation du di-N-propylacétonitrile.**

(30) Priorité : **15.09.87 FR 8712773**

(43) Date de publication de la demande : **22.03.89 Bulletin 89/12**

(45) Mention de la délivrance du brevet : **11.12.91 Bulletin 91/50**

(84) Etats contractants désignés : **AT BE CH DE ES FR GB GR IT LI LU NL SE**

(56) Documents cités : **US-A- 3 256 311**

(73) Titulaire : **SANOFI**
**40, Avenue George V**
**F-75008 Paris (FR)**

(72) Inventeur : **Bouisset, Michel**
**Lotissement Le Thor 73**
**F-04200 Sisteron (FR)**
Inventeur : **Forquy, Christian**
**Quartier Coos**
**F-64360 Monein (FR)**
Inventeur : **Bousquet, André**
**Rue de ma Vigne 27**
**F-04200 Sisteron (FR)**
Inventeur : **Heymes, Alain**
**Rue Frédéric Mistral 5**
**F-04200 Sisteron (FR)**

(74) Mandataire : **Le Guen, Gérard et al**
**CABINET LAVOIX 2, place d'Estienne d'Orves**
**F-75441 Paris Cédex 09 (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



Jouve, 18, rue Saint-Denis, 75001 PARIS

## Description

La présente invention se rapporte, d'une manière générale, à un nouveau procédé de préparation d'un dérivé d'acétonitrile.

En particulier, l'invention concerne un nouveau procédé pour la préparation du di-n-propylacétonitrile de formule :

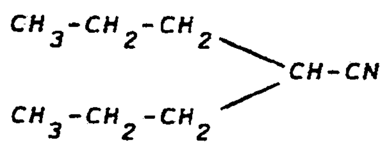

Le di-n-propylacétonitrile est un produit connu, particulièrement intéressant pour la préparation de composés possédant de précieuses propriétés pharmacologiques. Par exemple, le di-n-propylacétonitrile peut être mis en oeuvre pour la préparation du di-n-propylacétamide ou "valpromide" ou encore pour l'obtention de l'acide di-n-propylacétique ou "acide valproïque" tel que décrit dans les brevets FR-A-2383918 et FR-A-2383907.

Ces composés sont actuellement largement utilisés pour leurs propriétés neurotropes et spécialement pour leurs propriétés antiépileptiques.

Les procédés classiques pour préparer le di-n-propylacétonitrile sont généralement compliqués et nécessitent l'emploi de réactifs dangereux pour le personnel de fabrication. Par exemple, la préparation de di-n-propylacétonitrile à partir de di-n-propylcétone fait appel au cyanure de sodium, produit extrêmement toxique. De plus, certaines phases de préparation consistent en une hydrogénation toujours difficile à mener sur une grande échelle.

La recherche d'un procédé industriel pour l'obtention du di-n-propylacétonitrile, au départ, par exemple, de di-n-propylcétone reste donc d'un intérêt primordial.

On connaît déjà des procédés de préparation de nitriles aliphatiques à partir de dérivés de formamide par réaction de ceux-ci en phase gazeuse au contact de catalyseurs pour l'essentiel des catalyseurs à base de silice dopée ou non. Néanmoins, la plupart des documents antérieurs relatifs à ces procédés ne décrivent spécifiquement que la déshydratation de dérivés de formamide ne pouvant ni former des oléfines par rupture des liaisons C-N ni former des isomères.

A cet effet, on peut citer le brevet US-A-3256311 qui montre la préparation de nitriles aliphatiques à partir de formamides à une température comprise entre 460°C et 560°C au contact d'acide silicique actif ou de silicates éventuellement activés avec des oxydes métalliques des groupes III à VI de la classification périodique des éléments, par exemple l'oxide de titane. L'acide silicique actif peut en outre être combiné, le cas échéant, avec des oxydes basiques tels que l'oxyde de calcium, l'oxyde de magnésium, l'oxyde d'aluminium. Toutefois, cette combinaison ne présente aucune importance. La préparation de l'acétonitrile à partir du N-méthylformamide y est spécifiquement décrite, ce composé ne pouvant cependant former de produits secondaires de type isomérique ou éthylénique.

De même, on a rapporté la préparation de nitriles aliphatiques également à partir de formamides par réaction de déshydratation-réarrangement à 400-650°C en présence d'un catalyseur au phosphomolybdate de bismuth en présence de faibles quantités d'oxygène. Une telle réaction a été décrite dans le brevet FR-A-2341562 et son certificat d'addition FR-A-2418223.

Ces références enseignent essentiellement la déshydratation du n-éthylformamide en propionitrile mais n'évoquent absolument pas la formation de produits secondaires tel que l'éthylène.

Cependant, on peut relever que des essais effectués avec le N-tertiobutylformamide donnent de mauvais résultats en sélectivité.

Dans le cadre de l'élaboration de la présente invention, on a effectué des essais de préparation du di-n-propylacétonitrile par déshydratation du n-propyl-1 n-butylformamide selon les méthodes antérieures décrites ci-dessus, c'est-à-dire en mettant en oeuvre un catalyseur constitué de silice non dopée, de silice dopée au chlorure de titane ou encore d'un phosphomolybdate de bismuth.

Dans tous les cas, on a obtenu, suite à la rupture des liaisons C-N, des produits secondaires éthyléniques en quantité supérieure à 10% pouvant atteindre, dans certains cas, plus de 20%, ainsi que plus de 2% d'isomères du di-n-propylacétonitrile à savoir l'éthyl-2 hexanenitrile et le méthyl-2 heptanenitrile.

Par exemple, on a effectué la déshydratation du N-propyl-1 n-butylformamide en utilisant une silice non dopée comme catalyseur par application du procédé ci-dessous :

On place dans la partie médiane d'un réacteur tubulaire en verre pyrex de 200 $cm^3$ (diamètre 2 cm, longueur 32 cm), 13,5 $cm^3$ de gel de silice pastillée (2 × 3 mm) de caractéristiques suivantes :

| | |
|---|---|
| — surface spécifique : | 320 $m^2/g$ |
| — volume poreux : | 1,75 ml/g |
| — pH d'une suspension à 5% : | 6,0 |
| — teneur en $SiO_2$ : | 99,6% |
| — teneur en $Al_2O_3$ : | 0,15% |
| — teneur en $Na_2O$ : | 0,04% |

On place le réacteur dans un four électrique et on effectue un prétraitement du catalyseur pendant 20 heures sous flux d'azote (10 l/h) à 350°C puis on fait arriver, par le sommet du réacteur, 12 g (13,5 ml/h) de N-propyl-1 n-butylformamide et 10 l/h d'azote. On récupère en bas du réacteur les produits formés, par l'intermédiaire de deux récipients double enveloppe, l'un maintenu à 50°C et l'autre à −10°C. On effectue périodiquement l'analyse par chromatographie en phase gazeuse du mélange brut de produits obtenus. Après 7 heures de fonctionnement, on constate que la conversion du N-propyl-1 n-butylformamide et la sélectivité en produits exprimée en % par rapport au formamide converti n'évolue pas.

Le mélange brut a alors la composition suivante :

| | |
|---|---|
| - Heptènes | : 22,5 % |
| - Di-n-propylméthylamine | : 3,0 % |
| - Di-n-propylacétonitrile | : 66,5 % |
| - Ethyl-2 hexanenitrile | : 1,9 % |
| - Méthyl-2 heptanenitrile | : 0,5 % |
| - N-propyl-1 n-butylformamide non converti | : 5,6 % |

La conversion du N-propyl-1 n-butylformamide est donc de 94,4% pour un rendement de di-n-propylacétonitrile de 66,5%. En outre, on constate une production d'heptènes supérieures à 20% (double liaison en position 1 ou 2 minoritaire, en position 3 majoritaire) et d'isomères du di-n-propylacétonitrile d'environ 2,5%.

En conséquence, le procédé ainsi décrit ne peut être utilisé pour la préparation future de di-n-propylacétamide ou d'acide di-n-propylacétique, le di-n-propylacétonitrile obtenu étant souillé d'une quantité trop importante de produits secondaires. En effet, suivant les normes pharmaceutiques en vigueur, le di-n-propylacétamide ou l'acide di-n-propylacétique ne peut contenir plus de 0,4% d'impuretés.

On a maintenant trouvé, de manière imprévisible, qu'il est possible de préparer le di-n-propylacétonitrile selon un procédé continu en mettant en oeuvre la déshydratation à haute température du N-propyl-1 n-butylformamide en présence d'un catalyseur à base de silice dopée au moyen d'un élément basique, tout en réduisant considérablement la formation de produits secondaires.

Ainsi, on prépare selon l'invention le di-n-propylacétonitrile en portant à une température comprise entre 350°C et 550°C, préférentiellement entre 400°C et 500°C, et en l'absence d'oxygène, le N-propyl-1 n-butylformamide de formule :

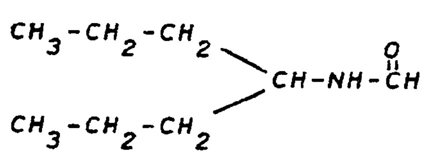

en présence d'un catalyseur constitué d'une silice imprégnée de 0,1 à 10% en poids, préférentiellement de 0,25 à 2% en poids de cations métalliques alcalins tels que par exemple $Na^+$ ou $K^+$, ce qui fournit le composé désiré.

Les catalyseurs utilisés dans le procédé de l'invention sont préparés à partir de gels de silice de surface spécifique comprise, de préférence, entre 200 et 500 $m^2/g$ et de volume poreux compris préférentiellement entre 0,8 et 2,0 ml/g.

Des gels de silice de ce type sont disponibles dans le commerce ou peuvent être préparés à partir de solutions aqueuses de silicate de sodium par précipitation avec l'ammoniaque.

Ces gels de silice sont alors imprégnés de cations métalliques alcalins, c'est-à-dire mis en contact, à température ambiante, avec une solution aqueuse d'un hydroxyde de métal alcalin ou d'un sel de métal alcalin tel que par exemple un carbonate de métal alcalin. Les gels de silice ainsi imprégnés peuvent alors, après séchage pendant 10 à 24 heures à 150-200°C, être mis en forme par extrusion ou pastillage selon des techniques usuelles.

La régénération du catalyseur en question après un temps de fonctionnement suffisamment long pour permettre de restaurer son activité initiale est réalisée par traitement sous oxygène dilué à une température permettant la combustion du coke formé. Un traitement de ce type restaure intégralement l'activité et la sélectivité de ce catalyseur.

La déshydratation catalytique du N-propyl-1 n-butylformamide s'effectue de préférence selon une réaction en phase gazeuse sur lit fixe de catalyseur à haute température, généralement 400 à 500°C. La mise en oeuvre d'une telle réaction nécessite, à ces températures, l'absence d'oxygène lequel conduit à des réactions successives de décomposition des molécules organiques présentes. Il est par conséquent nécessaire de conduire le procédé de l'invention sous gaz inerte tel qu'azote ou argon.

Les catalyseurs a base de gel de silice utilisés dans le procédé de l'invention présentent un intérêt considérable par le fait qu'ils permettent de préparer le di-n-propylacétonitrile avec des rendements de l'ordre de 80 à 85% tout en évitant la production d'heptènes et en ramenant la teneur en isomères du di-n-propylacétonitrile à une valeur inférieure à 0,1%.

En conséquence, un autre objet de l'invention concerne un catalyseur pour la mise en oeuvre du procédé selon l'invention constitué d'une silice imprégnée de 0,1% à 10% en poids de cations métalliques alcalins ou alcalino-terreux telle qu décrite ci-dessus.

Le N-propyl-1 n-butylformamide est un produit connu, décrit dans Chimie Thérapeutique, No 5 pp. 388-391 (1972) comme étant un composé difficile à préparer et de nature instable car évoluant dans le temps.

On a constaté, dans le cadre de la présente invention, que le N-propyl-1 n-butylformamide peut être préparé aisément par action à chaud par exemple à une température de 130 à 150°C d'un formiate d'alkyle en excès tel que le formiate d'éthyle, sur la di-n-propylméthylamine. On obtient de cette manière, un composé parfaitement défini et pur avec un rendement supérieur à 95%, ce composé étant stable à température ambiante.

Quant à la di-n-propylméthylamine, il s'agit également d'un composé connu d'après Chimie Thérapeutique cité précédemment. Cette amine peut être obtenue de manière particulièrement avantageuse par réaction du di-n-propylméthanol ou de la di-n-propylacétone avec l'ammoniac et l'hydrogène en présence d'un catalyseur au nickel par exemple le nickel de Raney, la réaction ayant lieu à une température comprise entre 150 et 180°C.

Selon ce procédé, on obtient un rendement en di-n-propylméthylamine supérieur à 95%.

Les Exemples non limitatifs suivants illustrent l'invention :

## EXEMPLE 1

### Préparation du di-n-propylacétonitrile

A) Di-n-propylméthylamine

On fait passer à travers un réacteur tabulaire (diamètre : 2,5 cm, longueur : 60 cm) contenant 50 $cm^3$ de catalyseur au nickel (55%) supporté sur Kieselgühr, un mélange constitué de di-n-propylcétone/ammoniac/hydrogène dans le rapport molaire 1/5/5 à une pression de 4,5 $10^5$Pa. Le débit horaire de di-n-propylcétone est de 25 $cm^3$/h et la température de régulation du four est de 175°C.

On récupère à la sortie du réacteur, l'effluent réactionnel dans un condenseur maintenu à 10°C.

L'analyse chromatographique du mélange montre qu'il est constitué de 97% de di-n-propylméthylamine, 1% de tri-(di-n-propylamino)-4 heptane et 1% de di-n-propylcétone.

B) N-Propyl-1 n-butylformamide

On fait passer à travers un réacteur tabulaire rempli de billes de verre (diamètre : 2,5 cm, longueur : 60 cm), un mélange constitué d'une mole de di-n-propylméthylamine et de quatre moles de formiate de méthyle, à un débit de 50 $cm^3$/h. La zone chauffée du réacteur correspond à 100 $cm^3$.

On maintient une température de 150°C dans cette partie du réacteur et l'on condense l'effluent réactionnel à la sortie du réacteur dans deux récipients en cascades, l'un maintenu à 35°C et l'autre à 0°C.

On obtient par analyse du premier condenseur un mélange de 98% de N-propyl-1 n-butylformamide et 2% de di-n-propylméthylamine.

Dans le deuxième condenseur, on récupère un mélange de méthanol et de formiate de méthyle.

C) di-n-propylacétonitrile

a) Catalyseur

On imprègne, de manière classique, 50 g de gel de silice de caractéristiques suivantes :

| | |
|---|---|
| — surface spécifique : | 320 $m^2/g$ |
| — volume poreux : | 1,75 ml/g |
| — pH d'une suspension à 5% : | 6,0 |
| — teneur en $SiO_2$ : | 99,6% |
| — teneur en $Al_2O_3$ : | 0,15% |
| — teneur en $Na_2O$ : | 0,04% |

dans une solution comprenant 10,8 ml d'hydroxyde de sodium 1N et 150 ml d'eau déminéralisée. On évapore la solution sous vide à 70°C et on place la silice imprégnée de 0,54 % de $Na^+$ ainsi obtenue, dans la partie médiane d'un réacteur tubulaire en pyrex de 200 $cm^3$ (diamètre 2 cm, longueur 32 cm). On place le réacteur dans un four électrique et on traite alors le catalyseur sous flux d'azote (10 l/h) pendant 20 heures à 350°C.

b) Nitrile

Par le sommet du réacteur on injecte alors à travers un diffuseur, 12 g (13,5 ml/h) de N-propyl-1 n-butylformamide et 10 l/h d'azote et on amène la température à 500°C. Après 7 heures de fonctionnement, on récupère le produit obtenu brut en bas du réacteur par l'intermédiaire de deux pots double enveloppe, l'un maintenu à 50°C et l'autre à −10°C. Durant ce fonctionnement, on effectue périodiquement l'analyse par chromatographie en phase gazeuse du mélange de produits bruts obtenus.

De cette manière, on obtient le di-n-propylacétonitrile sous forme brute, c'est-à-dire une composition contenant :

| | |
|---|---|
| - *Heptènes* | : 0 % |
| - *Di-n-propylméthylamine* | : 4,3 % |
| - *Di-n-propylacétonitrile* | : 78,3 % |
| - *Isomères du di-n-propylacétonitrile* | :< 0,1 % |
| - *N-Propyl-1 n-butylformamide non converti* | : 17,4 % |

Après la séparation par lavage acide de la di-n-propylméthylamine et distillation du di-n-propylacétonitrile, on peut récupérer la di-n-propylméthylamine pour restituer le formamide de départ et le N-propyl-1 n-butlyformamide non converti pour le recycler dans la réaction de déshydratation.

## EXEMPLE 2

### Préparation du di-n-propylacétonitrile

a) N-Propyl-1 n-butylformamide

Dans un réacteur de 1,5 l muni d'un système de chauffage, d'un agitateur, d'un thermomètre et d'un réfrigérant, on introduit sous balayage d'azote, 600 g (5,06 moles) de di-n-propylméthylamine et 520 g (7,01 moles) de formiate d'éthyle. On chauffe l'ensemble à 50°C durant 13 heures puis on concentre, à l'évaporateur rotatif, le produit brut de réaction de manière à éliminer l'excès de formiate d'éthyle et la di-n-propyméthylamine qui n'a pas réagi. On reprend avec 2000 ml d'éther diisopropylique le résidu ainsi obtenu et on le lave successivement avec 3 fois 200 ml d'acide chlorhydrique aqueux à 10% et 2 fois 200 ml d'eau. On sèche la phase organique sur sulfate de sodium et on concentre à l'évaporateur rotatif.

De cette manière, on obtient 656 g de N-propyl-1 n-butylformamide titrant à 98,6%.

Ce composé peut être obtenu analytiquement pur par distillation.

P.E. : 86°C (0,66 Pa)

*Analyse centésimale %.*

| | C | H | N |
|---|---|---|---|
| Calculé | 67,09 | 11,96 | 9,78 |
| Trouvé | 66,74 | 12,22 | 9,69 |

*Spectre I.R. (film) :*

| | | |
|---|---|---|
| NH associé | 3300 $cm^{-1}$ (m) | 3060 $cm^{-1}$ (m) |
| HC=O | 2860 $cm^{-1}$ (m) | |
| C=O | 1680 $cm^{-1}$ (f) | |

b) Di-n-propylacétonitrile

On utilise le procédé décrit à l'Exemple 1 en mettant en oeuvre un catalyseur à base du même gel de silice mais imprégné cette fois de 0,8% de $K^+$ apporté sous forme d'hydroxyde de potassium, préparé dans les mêmes conditions qu'à l'Exemple 1.

On obtient ainsi, après 12 heures de fonctionnement, le di-n-propylacétonitrile brut, à savoir une composition contenant :

- Heptènes : 0 %
- Di-n-propylméthylamine : 3,1 %
- Di-n-propylacétonitrile : 83,1 %
- Isomères du di-n-propylacétonitrile : < 0,1 %
- N-Propyl-1 n-butylformamide non converti : 13,8 %

EXEMPLE 3

Préparation du di-n-propylacétonitrile

On utilise le procédé décrit à l'Exemple 1 en mettant en oeuvre un catalyseur à base de gel de silice résultant de la précipitation du silicate de sodium. Ce gel, de surface spécifique égale à 260 $m^2/g$ et de volume poreux égale à 1,08 ml/g, est imprégné par la suite de 2,14% de $Na^+$.

On obtient ainsi à 450°C et après 92 heures de fonctionnement, le di-n-propylacétonitrile brut, à savoir la composition suivante :

- Heptènes : 2,1 %
- Di-n-propylméthylamine : 4,8 %
- Di-n-propylacétonitrile : 64,2 %
- Isomères du di-n-propylacétonitrile : < 0,1 %
- N-Propyl-1 n-butylformamide non converti : 28,8 %

On soumet alors le catalyseur à une régénération pendant 4 heures à 450°C sous un mélange gazeux composé de 99,5% d'azote et 0,5% d'oxygène à un débit de 13,5 l/h et 16 heures sous un mélange oxygène-/azote 1,5%/98,5%. Après ce traitement, on reprend l'injection du N-propyl-1 n-butylformamide dans l'azote et l'on obtient après 155 heures, le di-n-propylacétonitrile brut, c'est-à-dire une composition de formule :

- *Heptènes* : 0 %
- *Di-n-propylméthylamine* : 4,5 %
- *Di-n-propylacétonitrile* : 71,6 %
- *Isomères du di-propylacétonitrile* : < 0,1 %
- *N-Propyl-1 n-butylformamide non converti* : 23,9 %

On effectue alors une régénération du catalyseur suivant la même procédure que précédemment. Après reprise de l'injection des réactifs dans les mêmes conditions que précédemment (température : 450°C), on obtient, après 7 heures de fonctionnement, le di-n-propylacétonitrile brut à savoir la composition suivante :

- *Heptènes* : 0 %
- *Di-n-propylméthylamine* : 8,2 %
- *Di-n-propylacétonitrile* : 85,5 %
- *Isomères du di-n-propylacétonitrile* :< 0,1 %
- *N-Propyl-1 n-butylformamide non converti* : 6,2 %

## Revendications

1. Procédé de préparation du di-n-propylacétonitrile de formule :

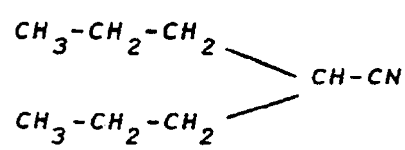

caractérisé en ce que l'on porte à une température comprise entre 350°C et 550°C, et en l'absence d'oxygène, le N-propyl-1 n-butylformamide de formule :

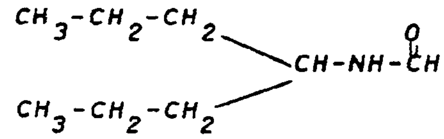

en présence d'un catalyseur constitué d'une silice imprégnée de 0,1 à 10% en poids de cations métalliques alcalins pour obtenir le composé désiré.

2. Procédé selon la revendication 1, caractérisé en ce que la température est comprise entre 400 et 500°C.

3. Procédé selon la revendication 1, caractérisé en ce que le catalyseur est obtenu par imprégnation d'un gel de silice ayant une surface spécifique comprise entre 200 et 500 $m^2/g$ et un volume poreux compris entre 0,8 et 2,0 ml/g.

4. Procédé selon la revendication 1 ou la revendication 3, caractérisé en ce que le catalyseur est obtenu par imprégnation d'un gel de silice avec une solution aqueuse d'un hydroxyde de métal alcalin ou un sel de métal alcalin.

5. Procédé selon la revendication 1, 3 ou 4 caractérisé en ce que la silice est imprégnée de 0,25 à 2% en poids de cations métalliques alcalins.

6. Procédé selon l'une quelconque des revendications 1, 3, 4 ou 5 caractérisé en ce que le métal alcalin

est le sodium ou le potassium.

7. Catalyseur pour la mise en oeuvre du procédé selon l'une quelconque des revendications 1 à 6, constitué de silice imprégnée de 0,1 à 10% en poids de cations métalliques alcalins.

8. Catalyseur selon la revendication 7, caractérisé en ce que la silice est imprégnée de 0,25 à 2% en poids de cations métalliques alcalins.

9. Catalyseur selon la revendication 7 ou la revendication 8, caractérisé en ce que le catalyseur est obtenu par imprégnation d'un gel de silice ayant une surface spécifique comprise entre 200 et 500 $m^2/g$ et un volume poreux compris entre 0,8 et 2,0 ml/g.

10. Catalyseur selon l'une quelconque des revendications 7 à 9, caractérisé en ce que le cation métallique alcalin est $Na^+$ ou $K^+$.

## Claims

1. Process for preparing di-n-propylacetonitrile of formula :

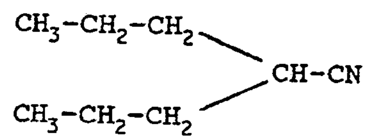

characterised in that N-(1-propyl-n-butyl)formamide of formula :

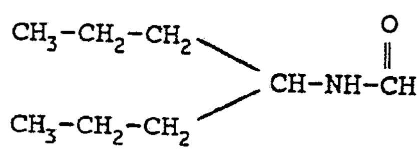

is heated to a temperature of between 350°C and 550°C, and in the absence of oxygen, in the presence of a catalyst consisting of a silica impregnated with 0.1 to 10% by weight of alkali metal cations, to obtain the desired compound.

2. Process according to claim 1, characterised in that the temperature is between 400 and 500°C.

3. Process according to claim 1, characterised in that the catalyst is obtained by the impregnation of a silica gel having a specific surface area of between 200 and 500 $m^2/g$ and a pore volume of between 0.8 and 2.0 ml/g.

4. Process according to claim 1 or claim 3, characterised in that the catalyst is obtained by the impregnation of a silica gel with an aqueous solution of an alkali metal hydroxide or an alkali metal salt.

5. Process according to claim 1, 3 or 4, characterised in that the silica is impregnated with 0.25 to 2% by weight of alkali metal cations.

6. Process according to any one of claims 1, 3, 4 or 5, characterised in that the alkali metal is sodium or potassium.

7. Catalyst for carrying out the process according to any one of claims 1 to 6, consisting of silica impregnated with 0.1 to 10% by weight of alkali metal cations.

8. Catalyst according to claim 7, characterised in that the silica is impregnated with 0.25 to 2% by weight of alkali metal cations.

9. Catalyst according to claim 7 or claim 8, characterised in that the catalyst is obtained by the impregnation of a silica gel having a specific surface area of between 200 and 500 $m^2/g$ and a pore volume of between 0.8 and 2.0 ml/g.

10. Catalyst according to any one of claims 7 to 9, characterised in that the alkali metal cation is $Na^+$ or $K^+$.

## Patentansprüche

1. Verfahren zur Herstellung von Di-n-propylacetonitril der Formel

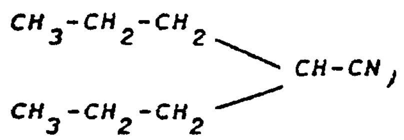

gekennzeichnet durch Erhitzen von N-(1-Propyl-n-butyl)-formamid der Formel

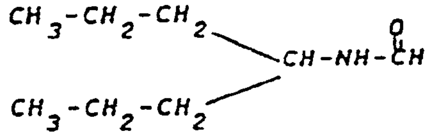

auf eine Temperatur zwischen 350 und 550 °C in Abwesenheit von Sauerstoff in Gegenwart eines Katalysators aus einer Kieselsäure, die mit 0,1 bis 10 Gew.-% an Alkalimetallkationen imprägniert ist, unter Erhalt der Zielverbindung.

2. Verfahren nach Anspruch 1, dadurch gekennzeichnet, daß die Temperatur zwischen 400 und 500 °C liegt

3. Verfahren nach Anspruch 1, dadurch gekennzeichnet, daß der Katalysator durch Imprägnierung eines Kieselsäuregels erhalten ist, das eine spezifische Oberfläche zwischen 200 und 500 $m^2/g$ und ein Porenvolumen zwischen 0,8 und 2,0 ml/g besitzt.

4. Verfahren nach Anspruch 1 oder Anspruch 3, dadurch gekennzeichnet, daß der Katalysator durch Imprägnierung eines Kieselsäuregels mit einer wäßrigen Lösung eines Alkalimetallhydroxids oder eines Alkalimetallsalzes erhalten ist.

5. Verfahren nach Anspruch 1, 3 oder 4, dadurch gekennzeichnet, daß die Kieselsäure mit 0,25 bis 2 Gew.-% Alkalimetallkationen imprägniert ist.

6. Verfahren nach einem der Ansprüche 1, 3, 4 oder 5, dadurch gekennzeichnet, daß das Alkalimetall Natrium oder Kalium ist.

7. Katalysator zur Durchführung des Verfahrens nach einem der Ansprüche 1 bis 6, bestehend aus mit 0,1 bis 10 Gew.-% Alkalimetallkationen imprägnierter Kieselsäure

8. Katalysator nach Anspruch 7, dadurch gekennzeichnet, daß die Kieselsäure mit 0,25 bis 2 Gew.-% Alkalimetallkationen imprägniert ist.

9. Katalysator nach Anspruch 7 oder 8, dadurch gekennzeichnet, daß durch Imprägnierung eines Kieselsäuregels mit einer spezifischen Oberfläche zwischen 200 und 500 $m^2/g$ und einem Porenvolumen zwischen 0,8 und 2,0 ml/g erhalten ist.

10. Katalysator nach einem der Ansprüche 7 bis 9, dadurch gekennzeichnet, daß das Alkalimetallkation $Na^+$ oder $K^+$ ist.